# FASCICULE DE BREVET EUROPEEN

(11) **EP 2 229 355 B1**
(45) Date de publication et mention de la délivrance du brevet: **19.09.2012**
(21) Numéro de dépôt: 08872601.3
(22) Date de dépôt: 12.12.2008
(51) Int. Cl.: C07D 205/04

(54) **PROCEDE DE PREPARATION DE DERIVES D ' AZETIDINE**
VERFAHREN ZUR HERSTELLUNG VON AZETIDINDERIVATEN
METHOD FOR PREPARING AZETIDINE DERIVATIVES

(30) Priorité: 14.12.2007 FR 0708746
(43) Date de publication de la demande: 22.09.2010
(73) Titulaire: SANOFI, 75008 Paris (FR)
(72) Inventeur: BIGOT, Antony, F-75013 Paris (FR); BOFFELLI, Philippe, F-75013 Paris (FR); LAMPILAS, Maxime, F-75013 Paris (FR); MAROLLEAU, Pascale, F-75013 Paris (FR); MEDARD, Alain, F-75013 Paris (FR); ODDON, Gilles, F-75013 Paris (FR); VARRAILLON, Daniel, F-75013 Paris (FR)
(74) Mandataire: Gaslonde, Aude
(86) Numéro de dépôt international: PCT/FR2008/001728
(87) Numéro de publication internationale: WO 2009/103883

(56) Documents cités:
- EP-A- 0 863 136
- WO-A-01/64634
- WO-A-2007/064566
- SAMMES P G ET AL: "ON THE SYNTHESIS OF AZETIDINES FROM 3-HYDROXYPROPYLAMINES" JOURNAL OF THE CHEMICAL SOCIETY, CHEMICAL COMMUNICATIONS, CHEMICAL SOCIETY. LETCHWORTH, GB, 1 janvier 1983 (1983-01-01), pages 682-684, XP008058218 ISSN: 0022-4936

## Description

La présente invention concerne un procédé de préparation de dérivés d'azétidine de type N-(1-benzhydryl-azetidin-3-yl)-N-phényl-méthylsulfonamide de formule (I) : 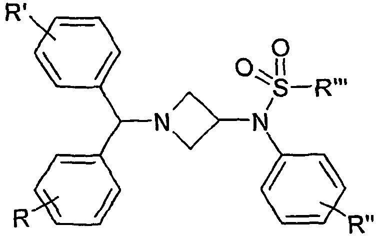 dans laquelle
R, R' et R" représentent, indépendamment l'un de l'autre, un ou plusieurs radicaux hydrogène, halogène (CI, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy .
R"' représente un groupe alkyle ou perfluoroalkyle linéaire ou ramifié de 1 à 6 atomes de carbone ou un groupement aryle éventuellement substitué par un ou plusieurs radicaux R".
Le procédé de synthèse des composés de formule (I) selon la présente invention est caractérisé en ce qu'il comprend les étapes suivantes : 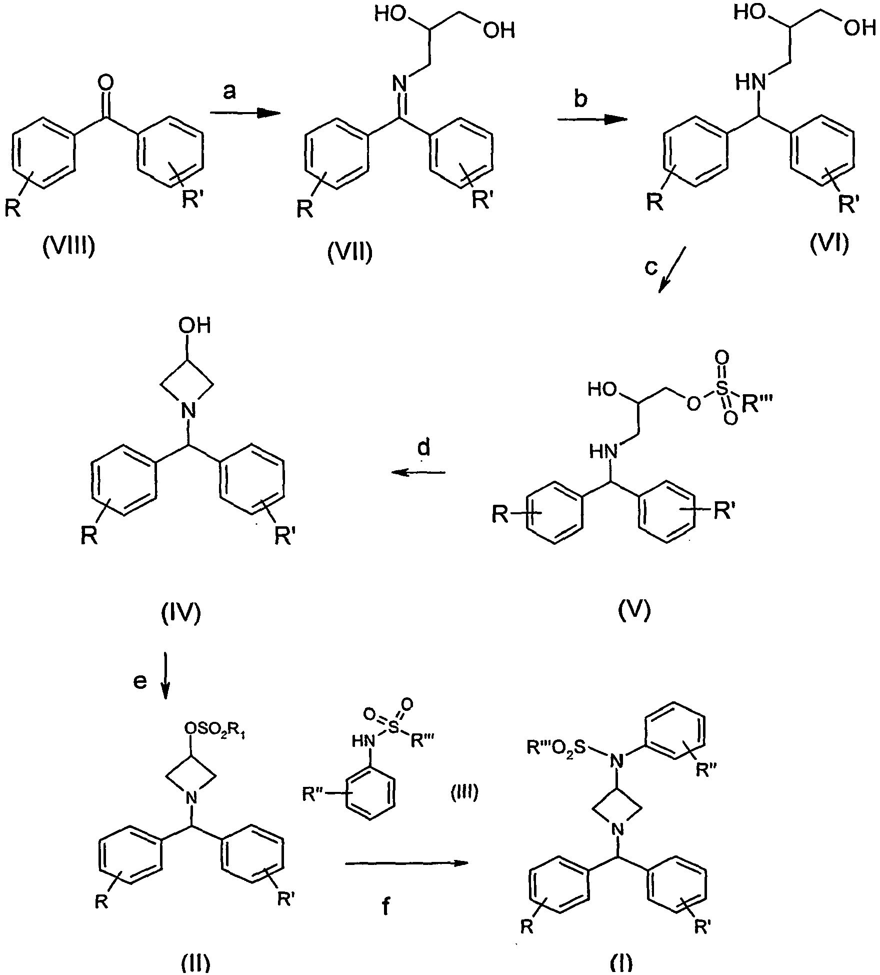
a) une cétone de formule (VIII) est condensée avec du 3-amino-1,2-propanediol, en présence ou non d'un solvant, en présence ou non d'un catalyseur acide ou basique, à une température comprise entre 0°C et 150°C ;
b) l'imine diol de formule (VII) obtenue à l'étape a) et éventuellement non isolée est réduite avec un hydroborure ou de l'hydrogène gazeux, en présence d'un catalyseur métallique dans un solvant pour former l'amine diol de formule (VI) ;
c) l'amine diol de formule (VI) obtenu à l'étape b) et éventuellement non isolé est activé par un agent sulfonylant en présence d'une base, dans un solvant et éventuellement un co-solvant à une température allant de -78°C à 40° pour former un ester sulfonique de formule (V) éventuellement non isolé ;
d) l'ester sulfonique de formule (V) obtenu à l'étape c) et éventuellement non isolé est cyclisé par chauffage dans un solvant en présence ou non d'une base, entre 0°C et 130°C pour former l'azétidinol de formule (IV) et est éventuellement isolé sous forme de sel ;
e) l'azétidinol de formule (IV) obtenu à l'étape d) est transformé en sulfonate de formule (II) par action d'un agent sulfonylant en présence d'une base, dans un solvant, à une température comprise entre -78° et +40°C ;
f) le sulfonate obtenu à l'étape e) est condensé avec un sulfonamide de formule (III) en présence d'une base, dans le même solvant que celui utilisé à l'étape e), pour former le composé de formule (I).

Dans les formules (I), (II), (III), (IV), (V), (VI), (VII) et (VIII), R , R' et R" représentent indépendamment l'un de l'autre, un ou plusieurs radicaux hydrogène, halogène (CI, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy ; R"' représente un groupe alkyle ou perfluoroalkyle linéaire ou ramifié de 1 à 6 atomes de carbone ou un groupement aryle éventuellement substitué par un ou plusieurs radicaux R" ; R1 représente un groupe méthyle, CF₃, C₄F₉, C₈F₁₇ ou phényle éventuellement substitué par un groupe méthyle, halogèno ou nitro.

Le document WO 0164634 décrit des voies d'accès générales pour l'obtention de tels produits et en particulier la synthèse des azétidinols de formule (IV) à partir du dérivé aminé et de l'épichlorhydrine ou épibromhydrine dans un solvant inerte. A une échelle industrielle, l'utilisation de tels réactifs peut présenter des risques pour la santé de ceux qui les manipulent, et devient très contraignante ; de plus la quantification de leurs traces résiduelles est extrêmement basse. Par conséquent la mise en place d'un procédé alternatif n'utilisant plus ce type de réactif devient importante. La présente invention permet de s'affranchir de l'utilisation de tels réactifs en maintenant une efficacité pour une production à large échelle.

La demande de brevet WO 0164634 décrit des voies d'accès générales pour l'obtention de produits de formule (I) et en particulier une synthèse par condensation d'un sulfonate et d'un sulfonamide, au sein d'un solvant inerte tel que le dioxanne, en présence de Cs₂CO₃ au reflux du mélange réactionnel. Les conditions opératoires pour la synthèse de composés de formule (I) telles qu'elles sont décrites dans le document WO 0164634, ne sont pas transposables à une échelle industrielle aussi bien en termes d'hygiène industrielle, de sécurité pour les manipulateurs et en termes de traces résiduelles des réactifs comme le Cs₂CO₃ et le dioxane. Ce nouveau procédé apporte des améliorations significatives en matière de sécurité, ainsi qu'une simplification des étapes de synthèse par l'utilisation d'un unique solvant dans les deux étapes. Les étapes de purifications, d'isolement d'intermédiaire et du produit fini, par chromatographie sur silice ont été supprimées et remplacées par des cristallisations, afin de rendre le procédé compatible avec une production industrielle.

Les étapes e) et f) sont réalisées dans le même solvant et sans agent de transfert de phase. Les conditions de synthèse doivent être compatibles avec une production à grande échelle et les traces résiduelles de solvants sans incidence. De plus, il a été trouvé que l'utilisation d'un même solvant dans les étapes e) et f) est possible.

On opère au sein d'un solvant compatible avec les contraintes des deux étapes, et il a été trouvé de manière surprenante qu'un solvant de type cétone, tel que la méthylisobutylcétone ou la méthyléthylcétone était compatible.
Le composé de formule (I) est obtenu après condensation du sulfonate de formule (II) avec un sulfonamide de formule (III), dans lesquelles R, R', R", R'" et R1 sont tels que précédemment définis. La formation du composé de formule (I) s'effectue par addition de sulfonamide de formule (III) au sulfonate de formule (II) dans des proportions molaires de (III)/(II) de 1 à 1,3 en présence d'une base, par exemple le carbonate de potassium, dans un solvant tel que la methyléthylcétone ou la methylisobutylcétone à une température entre 80 °C et 118°C. II a également été trouvé que l'absence de catalyseur dans cette étape de condensation favorise la diminution des impuretés.

Le sulfonate de formule (II), dans laquelle R1 est tel que précédemment défini, est obtenu à partir à partir de l'azétidinol de formule (IV), dans laquelle R et R' sont tels que précédemment définis, après réaction avec un agent sulfonylant. Cette réaction s'effectue en présence d'une base telle que la triéthylamine (TEA), la diisopropyléthylamine (DIPEA), le 1,5-diazabicyclo[4,3,0]non-5-ene (DBN), 1,8-diazabicyclo[5,4,0]undec-7-ene (DBU). La réaction s'effectue entre -78°C et +40°C.

Le sulfonamide de formule (III), dans laquelle R" et R"' sont tels que précédemment définis, est obtenu à partir de la 3,5-difluoroaniline, en présence d'un solvant tel que le THF (tétrahydrofurane) ou une cétone telle que la MiBUC (méthylisobutyl cétone) ou la MEC (méthyléthylcétone) et d'une base telle la triéthylamine (TEA), la diisopropyléthylamine (DIPEA), le 1,5-diazabicyclo[4,3,0]non-5-ene (DBN), 1,8-diazabicyclo[5,4,0]undec-7-ene (DBU).

L'azétidinol de formule (IV), dans laquelle R et R' sont tels que précédemment définis, est obtenu à partir du dérivé ester sulfonique, éventuellement non isolé, de formule (V), dans laquelle R et R' représentent, indépendamment l'un de l'autre, un ou plusieurs radicaux hydrogène, halogène (CI, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy et R"' représente un groupe alkyle ou perfluoroalkyle linéaire ou ramifié de 1 à 6 atomes de carbone ou un groupement aryle éventuellement substitué par un ou plusieurs radicaux R".

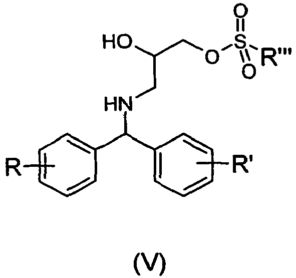

La formation de l'azétidinol de formule (IV) s'effectue par le chauffage de l'ester sulfonique de formule (V) dans un solvant, en présence ou non d'une base, et à une température comprise entre 0° et 130° C, par exemple entre 40°C et 110° C.

Par solvants, on entend les solvants aromatiques tels que toluène, xylène, chlorobenzène, les éthers tels que THF (Tetrahydrofurane), DME (Diméthoxyéthane), MTBE (Méthyltert-butyl éther) ou dioxane, les solvants chlorés tels que le dichlorométhane, le chloroforme, le 1,2-dichloroéthane, les esters tels que l'acétate d'éthyle ou de butyle, les nitriles tel que l'acétonitrile, les cétones telles que l'acétone, la méthyléthylcétone, la méthylisobutylcétone, les amides tels que le DMF (Diméthylformamide), la DMAC (Diméthylacétamide), la NMP (N-méthylpyrrolidone), les alcools tels que le méthanol, l'éthanol, l'isopropanol ou le butanol.

Par base, on entend les amines (triéthylamine, diisopropyléthylamine ...), les hydrogénocarbonates, les carbonates, les hydroxydes, les phosphates de métaux alcalins tels que lithium, sodium, potassium, césium.

Un autre mode de réalisation consiste à opérer dans le toluène à chaud à 80°C en présence d'une base inorganique, en particulier le NaHCO₃ (hydrogénocarbonate de sodium) ou Na₂CO₃ (carbonate de sodium).

Le dérivé de formule (IV) peut être isolé, purifié sous forme de sel de HX, X étant un CI, Br ou BF₄.

Le dérivé de formule (V), dans laquelle R et R' sont tels que précédemment définis, est synthétisé à partir d'une amine diol de formule (VI), dans laquelle R et R' représentent indépendamment l'un de l'autre un ou plusieurs radicaux hydrogène, halogène (CI, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy. 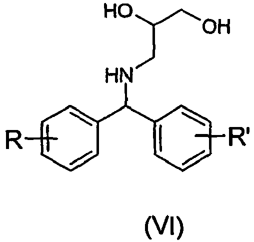 La formation de l'ester sulfonique de formule (V) s'effectue par l'addition d'un agent sulfonylant à l'amine diol (VI), en présence d'une base, dans un solvant et à une température comprise entre - 78° C et + 40° C, par exemple entre -30° C et 20° C. II est également possible d'ajouter une quantité catalytique (de 1 à 100 % molaire par rapport à l'amine de formule (VI)) d'une pyridine substituée telle que la 4-N,N-diméthylaminopyridine (DMAP).

Par agent sulfonylant, on entend les halogénures (fluorures, chlorures, bromures) d'alkyle (méthyl, éthyl, trifluorométhyl ...) ou d'aryle (phényl, para-méthylphényl...) sulfonyle, ou bien les anhydrides sulfoniques correspondants. L'agent sulfonylant est utilisé dans des proportions allant de 1 à 3,5 équivalents par rapport à l'amine diol (VI).

Par base, on entend les amines (triéthylamine, diisopropyléthylamine, ...), la pyridine (substituée ou non par des groupements alkyles), les hydrogénocarbonates, les carbonates, les hydroxydes, les phosphates de métaux alcalins lithium, sodium, potassium, césium.

Les solvants utilisables seuls ou en combinaison sont les solvants aromatiques tels que toluène, xylène, chlorobenzène, pyridine, les éthers tels que THF, DME, MTBE ou dioxane, les solvants chlorés tels que le dichlorométhane, le chloroforme, le 1,2-dichloroéthane, les esters tels que l'acétate d'éthyle ou de butyle, les nitriles tel que l'acétonitrile, les cétones telles que l'acétone, la méthyl isobutylcétone, les amides tels que le DMF, la DMAC, la NMP.

Un autre mode de réalisation consiste à opérer dans de la pyridine contenant éventuellement du toluène en présence de chlorure de para-toluènesulfonyle à une température allant de -20°C à 20°C.

Un autre mode de réalisation consiste à opérer dans du toluène en présence d'un co-solvant tel que la pyridine dans des proportions pyridine : toluène allant de 1 :1 à 1 : 4 en présence de chlorure de para-toluènesulfonyle à une température allant de -30°C à 20°C.

Le dérivé amine diol de formule (VI), dans laquelle R et R' sont tels que précédemment définis, est obtenu par réduction à partir d'une imine diol, éventuellement non isolée, de formule (VII) dans laquelle R et R' représentent, indépendamment l'un de l'autre, un ou plusieurs radicaux hydrogène, halogène (CI, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy.

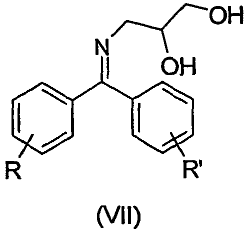

Les agents réducteurs utilisés peuvent être des hydroborures, tels que borohydrure de lithium, borohydrure de sodium, borohydrure de potassium, borohydrure de magnésium, borohydrure de calcium, et borohydrure de zinc ; des complexes du borane (borane-THF, borane-diméthylsulfide, amines-boranes) ; de l'hydrogène gazeux en présence de catalyseurs métalliques (palladium sur charbon, nickel de Raney). Les agents réducteurs sont utilisés dans des proportions allant de 1 à 10 équivalents par rapport à l'imine (VII). En fonction de la compatibilité avec les réducteurs utilisés, les solvants potentiellement utilisables sont les solvants aromatiques tels que toluène, xylène, chlorobenzène, les éthers tels que THF, DME, MTBE ou dioxane, les solvants chlorés tels que le dichlorométhane, le chloroforme, le 1,2-dichloroéthane, les esters tels que l'acétate d'éthyle ou de butyle, les alcools tels que le méthanol, l'éthanol, l'isopropanol ou le butanol, ou l'eau.

Un autre mode de réalisation consiste à effectuer la réduction en présence de NaBH₄ dans un mélange eau/ éthanol à 60°C.

Le dérivé imine diol de formule (VII) est synthétisé à partir du 3-amino-1,2-propanediol et d'une cétone de formule (VIII), dans laquelle R et R' représentent indépendamment l'un de l'autre un ou plusieurs radicaux hydrogène, halogène (CI, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy.

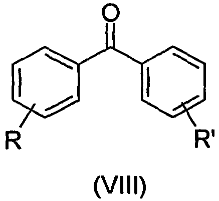

La formation de l'imine diol de formule (VII) s'effectue par chauffage, en présence ou en l'absence de solvant, à une température comprise entre 0 et 150°C et éventuellement en présence d'une catalyse acide ou basique ou d'un agent déshydratant. La déshydratation du milieu peut être éventuellement effectuée par distillation azéotropique. Les solvants potentiellement utilisables sont les solvants aromatiques tels que toluène, xylène, chlorobenzène, trifluorométhylbenzène, les éthers tels que THF, DME, MTBE ou dioxane, les solvants chlorés tels que le dichlorométhane, le chloroforme, le 1,2-dichloroéthane, les esters tels que l'acétate d'éthyle ou de butyle, les alcools tels que le méthanol, l'éthanol, l'isopropanol ou le butanol. Le catalyseur acide ou basique est utilisé dans des proportions variant de 1 % à 100 % molaire par rapport à la cétone.

Par catalyseur acide, on entend les acides chlorhydrique, bromhydrique, iodhydrique, l'acide tétrafluoroborique, l'acide hexafluorophosphinique, l'acide sulfurique, les acides sulfoniques tels que l'acide méthane sulfonique, p-toluène sulfonique, camphre sulfonique, trifluorométhane sulfonique, les acides sulfoniques supportés sur résine, les acides carboxyliques tels que l'acide oxalique, l'acide formique, les acides de Lewis tels que l'éthérate de trifluorure de bore, les triflates de terre rare.

Par agent déshydratant on entend le sulfate de magnésium ou de sodium, ou l'utilisation de tamis moléculaire.

Par base, on entend les hydrogénocarbonates, les carbonates, les hydroxydes, les phosphates de métaux alcalins lithium, sodium, potassium, césium. Le 3-aminopropane-1,2-diol est utilisé dans des proportions variant de 1 à 10 équivalents molaires par rapport à la cétone.

Un autre mode de réalisation consiste à opérer dans du xylène à 140°C en catalyse acide à 29% d'acide para-toluènesulfonique.

Par rapport au document WO 0164634, ce nouveau procédé apporte des améliorations en matière de sécurité, ainsi qu'une simplification des étapes de purification et d'isolement des intermédiaires ou du produit fini, notamment par élimination de l'utilisation de réactifs nocifs pour ceux qui les manipulent, afin de rendre le procédé compatible avec une production industrielle.
A titre d'exemple, le schéma réactionnel ci-dessous illustre de manière non limitative les réactifs utilisés pour chacune des étapes. Dans ce schéma, R et R' représentent des atomes de chlore en position para, R" représente un atome de fluor, R"' et R1 représentent un groupe méthyle.
La mise en réaction de la (4,4')-dichlorobenzophénone et du 3-aminopropane-1,2-diol dans des conditions classiques (catalyse acide par 29% d'acide para-toluènesulfonique sous Dean-Stark) permet d'obtenir l'imine désirée avec un rendement quasi quantitatif. Ce produit n'est pas isolé mais est directement réduit par l'action du NaBH₄ dans un mélange éthanol/eau à 60° C, pour conduire à l'aminodiol correspondant avec un rendement de 90 % pour 2 étapes. 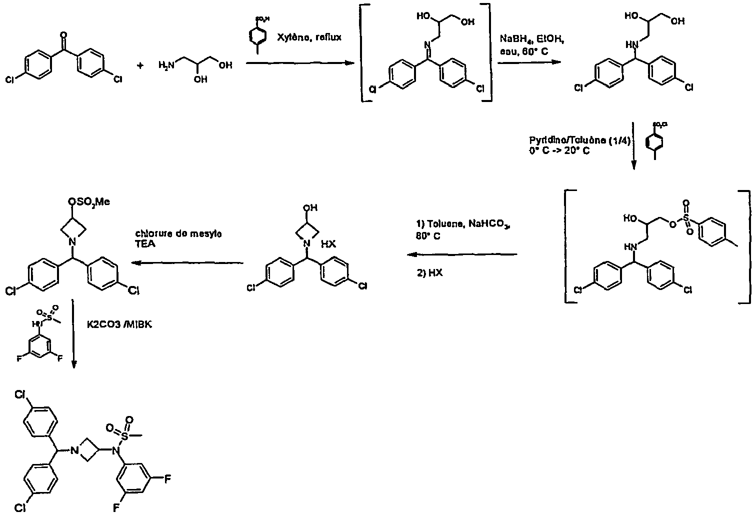 L'aminodiol peut être engagé dans l'étape suivante sans isolement ou bien être isolé par cristallisation dans le toluène ou bien dans un mélange de solvant avec le toluène. Parmi les solvants utilisés, on peut citer l'heptane, le cyclohexane ou le méthyl cyclohexane.
Cet aminodiol, mis en présence de chlorure de para-toluènesulfonyle dans un mélange Pyridine/Toluène (de 1/1 à 1/4) d'abord à -15° C, puis en laissant remonter la température aux alentours de 20° C, conduit chimiosélectivement au monotosylate intermédiaire qui n'est pas isolé, mais cyclisé directement dans le toluène à chaud (80° C) en présence d'une base inorganique (NaHCO₃) pendant 4 heures.
Après lavage de la phase organique par de l'eau, le produit est purifié par cristallisation sous forme de bromhydrate, pour conduire au bromhydrate de 1-[bis-(4-chlorophényl)méthyl]azétidin-3-ol avec un rendement global de 63 % pour 4 étapes.

### Partie expérimentale pour la synthèse du bromhydrate, chlorhydrate et tetrafluoroborate de 1-[bis-(4-chlorophényl)méthyl]azétidin-3-ol

### Etapes 1 et 2

Dans un réacteur de 1,5 litres muni d'une agitation mécanique, d'une sonde thermométrique, placé sous atmosphère d'azote, on introduit à une température de 20 ± 2°C : 50 g de 4,4' dichlorobenzophénone, 500 mL de xylène et une solution contenant 54,5 g de 3-amino propane-1,2-diol dans 78,5 ml d'éthanol. L'éthanol est alors éliminé par distillation en chauffant la solution ainsi obtenue jusqu'à ce que la température en tête de colonne atteigne 140 ± 2°C. La température de la masse réactionnelle est alors ramenée à environ 60 ± 2°C, puis du xylène est ajouté de manière à compléter le volume jusqu'à 500 mL. 10,97 g d'acide para-toluènesulfonique sont alors ajoutés, puis la masse réactionnelle est portée à une température de 140 ± 2°C tout en réalisant une distillation à volume constant pendant 13 heures. La masse réactionnelle contenant le 3-{[Bis-(4-chloro-phenyl)-méthylène]-amino}-propane-1,2-diol est alors concentrée à 3,25 volumes par distillation, puis la température est ramenée à environ 20 ± 2°C. 250 ml d'EtOH sont alors ajoutés, puis la solution est portée à 60 ± 2°C, puis traitée en 60 minutes environ par 151 ml (1 mole par mole de produit) de solution d'hydroborure de sodium (solution préparée extemporanément à partir de 8 g d'hydroborure de sodium, 0,850 ml de lessive de soude à 32 % et 150 ml d'eau déminéralisée). II est entendu que le 3-{[Bis-(4-chloro-phenyl)-methylene]-amino}-propane-1,2-diol peut être éventuellement isolé après distillation et lavage.

La solution obtenue est maintenue sous agitation pendant 5 heures à 60 ± 2°C. La masse réactionnelle est alors ramenée à 20 ± 2° C, puis traitée par 387 ml d'acide chlorhydrique 2N en 60 minutes environ à 20°/22°C. Le milieu réactionnel est alors maintenu sous agitation pendant 2 heures. La masse réactionnelle est alors chauffée jusqu'à atteindre 102° C tout en distillant le solvant, puis ramenée à 60 ± 2 C°. 250 ml de xylène sont ajoutés tout en continuant de refroidir le milieu réactionnel. Lorsque celui-ci atteint 20 ± 2° C, une quantité de soude 2N suffisante pour atteindre une valeur de pH comprise entre 8 et 9 est ajoutée (soit environ 150 ml). La phase aqueuse est décantée et la phase organique est traitée, sous agitation, par 700 ml d'eau déminéralisée puis en 10 minutes environ, par une quantité d'HCl 2N suffisante pour atteindre une valeur de pH comprise entre 1 et 2 (soit environ 160 ml). La phase aqueuse est décantée, récupérée, puis sont ajoutés 400 ml de toluène, puis le mélange est traité sous agitation en 10 minutes environ par une quantité de soude 2N suffisante pour atteindre une valeur de pH comprise entre 10 et 11 (soit environ 190 ml), tout en maintenant la température du milieu à environ 20 ± 2° C. Après 30 minutes d'agitation, la phase organique est décantée et la phase aqueuse est extraite par 100 ml de Toluène. Les phases organiques sont réunies, lavées par 200 ml d'eau déminéralisée, décantées, et cette opération est répétée 2 fois supplémentaires. La phase toluénique ainsi obtenue est alors deshydratée par distillation à volume constant et à pression atmosphérique d'environ 400 ml de toluène. On obtient ainsi 510 g d'une solution toluénique de 3-{[bis-(4-chloro-phényl)-methyl]-amino}-propane-1,2-diol. Cette solution est engagée telle quelle dans l'étape suivante.

Le 3-{[bis-(4-chloro-phényl)-methyl]-amino}-propane-1,2-diol peut être isolé par concentration de la solution toluènique à 2 volumes à 50°C puis additionnée de 2 volumes de cyclométhylcyclohexane et refroidi à 0°C. Le produit est essoré et lavé avec du cyclométhylcyclohexane à 0°C, séché en étuve à 40°C. Le rendement est de 86%.
Somme des impuretés HPLC : 0,4%m/m

### CARACTERISTIQUES:

Spectre RMN 1H à 500 MHz, DMSO-d6 référencé à 2,50 ppm à la température de 303K:
2.33-2.40(m,2H), 2.51(m,1H), 3.32(m,2H), 3.58(m,1H), 4.44(t,J=5.4Hz,1H), 4.58(d,4.9Hz,1H), 4.83(s,1H), 7.35(d,J=8.3Hz,4H), 7.42(d,J=8.3Hz,4H).
Spectre RMN 13C à 500 MHz, DMSO-d6 référencé à 39.5 ppm à la température de 303K: 51.0, 64.4, 64.9, 70.4, 128.3, 128.8, 131.2, 143.2.
Spectre de masse : m/z = 325Da (M+°).
Spectre IR : KBr 3331; 2909; 2829; 1595; 1489; 1410; 1088; 1014; 813; 530; 504

### Etapes 3 et 4

400 ml d'une solution toluénique contenant 44,17 g de 3-{[bis-(4-chloro-phényl)-methyl]-amino}-propane-1,2-diol, préparé comme précédemment, sont placés dans un réacteur de 1,5 L, muni d'une agitation mécanique, d'une sonde thermométrique, sous atmosphère d'azote, puis traités par 100 ml de pyridine. La solution ainsi obtenue est refroidie à - 7 ± 2° C, puis traitée par portions en 5 minutes environ par 27,2 g de chlorure de para-toluènesulfonyle. Après l'addition, le milieu réactionnel est agité pendant environ 1 heure à -7 ± 2° C, remonté à environ 20 ± 2° C en environ 1 heure, puis agité à environ 20 ± 2° C pendant environ 18 heures. Le milieu réactionnel est alors lavé par 200 ml d'eau déminéralisée, la phase organique est décantée, puis sont ajoutés 100 ml d'eau déminéralisée et le pH du milieu réactionnel est amené à environ 3 par l'ajout d'HCl 37 %. La masse réactionnelle est agitée pendant environ 10 minutes, la phase organique est décantée, récupérée, puis lavée par 100 ml d'eau déminéralisée. La masse réactionnelle est agitée pendant environ 10 minutes, la phase organique est décantée, récupérée, puis lavée par une quantité suffisante d'une solution aqueuse à 5 % de NaHCO₃ pour atteindre une valeur de pH supérieure à 5. La masse réactionnelle est agitée pendant environ 10 minutes, la phase organique est décantée, récupérée, puis lavée par 200 ml d'eau déminéralisée. La masse réactionnelle est agitée pendant environ 10 minutes, la phase organique contenant le toluène-4-sulfonique acide 3-{[bis-(4-chloro-phenyl)-methyl]-amino}-2-hydroxy-propyl ester est décantée, récupérée, puis traitée par 17,1 g de NaHCO₃. Le milieu réactionnel est chauffé à une température voisine de 79 ± 2° C, et laissé à cette température pendant 4 heures environ. La température de la masse réactionnelle est alors ramenée à environ 20 ± 2° C et la masse est lavée par trois fois 250 ml d'eau déminéralisée. Sur la solution toluénique ainsi obtenue, sont alors ajoutés en 1 heure environ 16 ml d'une solution d'acide bromhydrique à 48 % dans l'eau. La suspension est maintenue à 20 ± 2° C pendant environ 1 heure, puis le solide est filtré, lavé par trois fois 200 ml de toluène, puis séché 15 heures à 48 ± 2° C sous un vide d'environ 20 mbars.
Il est entendu que le toluène-4-sulfonique acide 3-{[bis-(4-chioro-phenyl)-methyl]-amino}-2-hydroxy-propyl ester peut être isolé.

On obtient ainsi 31,5 g de bromhydrate de 1-[bis-(4-chlorophényl)méthyl]azétidin-3-ol avec un rendement de 60 %.

### CARACTERISTIQUES

Spectre RMN 1H à 500 MHz, DMSO-d6 référencé à 2,50 ppm à la température de 303K: 3.80-4.30(m,4H), 4.50(m,0.7H), 4.63(m,0.3H), 5.99(m,1H), 6.24(m, 1H), 7.50-7.65(m,8H), 10.07(m,0.7H), 11.41 (m,0.3H).
Spectre de masse : m/z = 307Da (M+°).
Spectre IR: KBr 3342; 2901; 2787; 2630; 2589; 2423; 1600; 1495; 1425; 1140; 1093; 1014; 815; 533; 504cm⁻¹.

Un autre mode opératoire consiste à isoler le produit sous forme de chlorhydrate en coulant sur la solution toluénique de 1-[bis-(4-chlorophényl)méthyl]azétidin-3-ol obtenue comme ci-dessus une solution aqueuse d'HCl.

On obtient ainsi le chlorhydrate de 1-[bis-(4-chlorophényl)méthyl]azétidin-3-ol.

50 g de 3-{[bis-(4-chloro-phényl)-methyl]-amino}propane-1,2-diol, préparés et isolés comme précédemment, sont placés dans un réacteur de 1 L, muni d'une agitation mécanique, d'une sonde thermométrique, sous atmosphère d'azote, puis traitée par 150 ml de pyridine et 150ml de toluène. La solution ainsi obtenue est refroidie à - 15 ± 2° C, puis traitée par portions en 5 minutes environ par 73 g de chlorure de *para*-toluènesulfonyle en solution dans 125 ml de pyridine et 125 ml de toluène. Après l'addition, le milieu réactionnel est agité pendant environ 3 heures à -15 ± 2° C, remonté à environ 20 ± 2° C en environ 1 heure, puis le milieu réactionnel est alors lavé par 225 ml d'eau déminéralisée, la phase organique est décantée, puis sont ajoutés 250 ml de dichlorométhane. La phase organique est alors lavée par ajout de 410 ml d'HCl 37 % dilué au ½. La masse réactionnelle est agitée pendant environ 10 minutes, la phase organique est décantée, récupérée, puis lavée par 100 ml d'eau déminéralisée puis lavée par une quantité suffisante d'une solution aqueuse à 5 % de NaHCO₃ pour atteindre une valeur de pH supérieure à 5. La phase organique contenant le toluène-4-sulfonique acide 3-{[bis-(4-chloro-phenyl)-methyl]-amino}-2-hydroxy-propyl ester est concentrée à 275 ml puis distillée à volume constant par ajout de toluène pour éliminer le dichlorométhane, puis traitée par 19,3 g de NaHCO₃. Le milieu réactionnel est chauffé à une température voisine de 79 ± 2° C, et laissé à cette température pendant 4 heures environ. La température de la masse réactionnelle est alors ramenée à environ 20 ± 2° C et la masse est lavée par 250 ml d'eau déminéralisée. Sur la solution toluénique ainsi obtenue, sont alors ajoutés en 1 heure environ 13,5 ml d'une solution d'acide chlorhydrique 12N. La suspension est maintenue à 20 ± 2° C pendant environ 5 heures, puis le solide est filtré, lavé par trois fois 100 ml de toluène, puis séché 15 heures à 48 ± 2° C sous un vide d'environ 20 mbars.

On obtient ainsi 43,1 g de chlorhydrate de 1-[bis-(4-chlorophényl)méthyl]azétidin-3-ol avec un rendement de 81,7 %.

### CARACTERISTIQUES

Spectre RMN 1H à 400 MHz : (DMSO-d6) référencé à 2,50 ppm à la température de 303K : 3,70 à 4,20 (m, 4H) ; 4,47 (m, 0,8H) ; 4,68 (m, 0,2H) ; 5,97 (m, 1H) ; 6,34 (m étalé, 1H) ; 7,50 (d, J = 8,5 Hz, 4H) ; de 7,62 à 7,77 (m, 4H) ; 12,35 (m étalé, 0,2H) ; 12,5 (m étalé, 0,2H).

Spectre de masse : m/z=307 (M⁺).

Spectre IR : KBr 3289; 2753; 2583; 2448; 1596; 1495; 1458; 1425; 1142; 1091; 1014; 815; 801 & 534 cm⁻¹

Un autre mode opératoire consiste à isoler le produit sous forme de tétrafluoroborate en coulant sur la solution toluénique de 1-[bis-(4-chlorophényl)méthyl]azétidin-3-ol obtenue comme ci-dessus une solution aqueuse de HBF₄ à 50 % en 1 heure environ. La suspension est maintenue à 20 ± 2° C pendant environ 1 heure, puis le solide est filtré, lavé par trois fois 200 ml de toluène, puis séché 15 heures à 48 ± 2° C sous un vide d'environ 20 mbars.

On obtient ainsi le tétrafluoroborate de 1-[bis-(4-chlorophényl)méthyl]azétidin-3-ol.

Spectre RMN 1H à 400 MHz : (DMSO-d6) référencé à 2,50 ppm à la température de 303K : 3,85 (m, 2H) ; 4,25 (m, 2H) ; 4,50 (m, 0,6H) ; 4,58 (m, 0,4H) ; 5,87 (m, 1H) ; 6,20 (m très étalé, 1H) ; 7,51 (d, J = 9,0 Hz, 4H) ; 7,55 (d, J = 9,0 Hz, 4H) ; 10,7 (m étalé, 0,6H) ; 11,35 (m étalé, 0,4H)

Spectre IR : KBr 3528; 3202; 2937; 2785; 2620; 1597; 1496; 1142; 1093; 1061; 1038; 1013; 803 & 530 cm⁻¹

Spectre de masse : m/z=307(M - H)⁺.

### Etape 5

Dans un réacteur de 1,5 litres muni d'une ampoule de coulée, d'une agitation mécanique, d'une sonde thermométrique, et placé sous atmosphère d'azote sont introduits 40 g (102 mmoles) de bromhydrate de 1-[bis-(4-chlorophényl)méthyl]azétidin-3-ol, puis 320 ml de méthylisobutyl cétone (MIBK) à 20±2°C. On agite pendant 30 minutes, puis en 20 minutes, on ajoute 44 mL de triéthylamine tout en faisant descendre la température jusqu'à -5°C. On rince l'ampoule avec 40 mL de méthylisobutyl cétone. Le milieu est refroidi à -10° ± 2°C et le chlorure de méthane sulfonyle (12 ml, 153 mmoles) est ajouté au goutte à goutte en 30 minutes environ tout en maintenant la température à -10° ± 2°C. La suspension blanche est maintenue sous agitation pendant 1 heure à -12° ± 2°C. Le tout est ramené à 20° ± 2°C, puis agité pendant 1 heure. On ajoute 160 mL d'une solution à 50 g/l d'hydrogénocarbonate de sodium en 10 minutes environ. La phase organique devient limpide et la phase aqueuse est trouble. On maintient l'agitation pendant 30 minutes, puis on laisse décanter le tout, puis on lave la phase organique avec 2x80 mL d'une solution à 50 g/l d'hydrogénocarbonate de sodium. On obtient 2 phases limpides. Après élimination de la phase aqueuse, on récupère 400 ml d'une solution de mésylate d'azétidinol dans la MIBK, utilisée telle quelle pour la réaction suivante.

### Etape 6

A la solution de 400 mL de méthylisobutyl cétone contenant le méthylsulfonate de 1-[bis(4-chlorophényl)méthyl]azétidin-3-yle, on ajoute 21,37 g (105 mmol) de N-(3,5-difluorophényl)méthylsulfonamide puis 42,4 g de carbonate de potasium anhydre. Le tout est porté au reflux à une température de 105°C et agité à cette température pendant 4 heures. Puis, on ramène la température à 25°C en 30 minutes et on ajoute 200 mL de méthylisobutyl cétone, puis 200 mL d'eau. On agite pendant 15 minutes. Le milieu est décanté et la phase organique est lavée avec 200 mL d'eau pendant 15 minutes. De nouveau, on laisse décanter le tout.

La phase organique est alors concentrée par distillation d'environ 500 mL de MIBK sous agitation, sous un vide d'environ 23 mbars et à une température d'environ 24°C ± 2°C. On précipite le N-(1-[bis-(4-chlorophényl)méthyl]azétidin-3-yl)-N-(3,5-difluorophényl)-méthylsulfonamide en ajoutant au milieu 480 mL d'isopropanol à 25 °C ± 2°C. On abaisse la température à -3°C en 15 minutes environ et on agite pendant 14 heures à cette température.

La suspension est ensuite filtrée sur un verre fritté. Le réacteur est lavé 2 fois avec 80 mL d'isopropanol à 3°C, puis le gâteau ainsi obtenu est lavé à partir des fractions d'isopropanol récupérées.
On obtient une masse avant séchage de 53,94 g. Après séchage pendant 24 heures sous vide d'environ 40 mbars, et à une température de 40°C, on obtient 39,34 g de N-{1-[bis-(4-chlorophényl)méthyl]azétidin-3-yl}-N-(3,5-difluorophényl)-méthylsulfonamide, soit un rendement de 77,4%.

Les conditions décrites dans l'exemple précédent (étapes 5 et 6) ont été appliquées dans des conditions similaires au départ du chlorhydrate de 1-[bis-(4chlorophényl)méthyl]azétidin-3-ol et ont conduit à l'obtention du N-{1-[bis-(4-chlorophényl)méthyl]azétidin-3-yl}-N-(3,5-difluorophényl)-méthylsulfonamide avec un rendement de 76,7%

## Revendications

1. Procédé de synthèse de dérivés d'azétidine de formule (I), **caractérisé en ce qu'**il comprend les étapes suivantes : 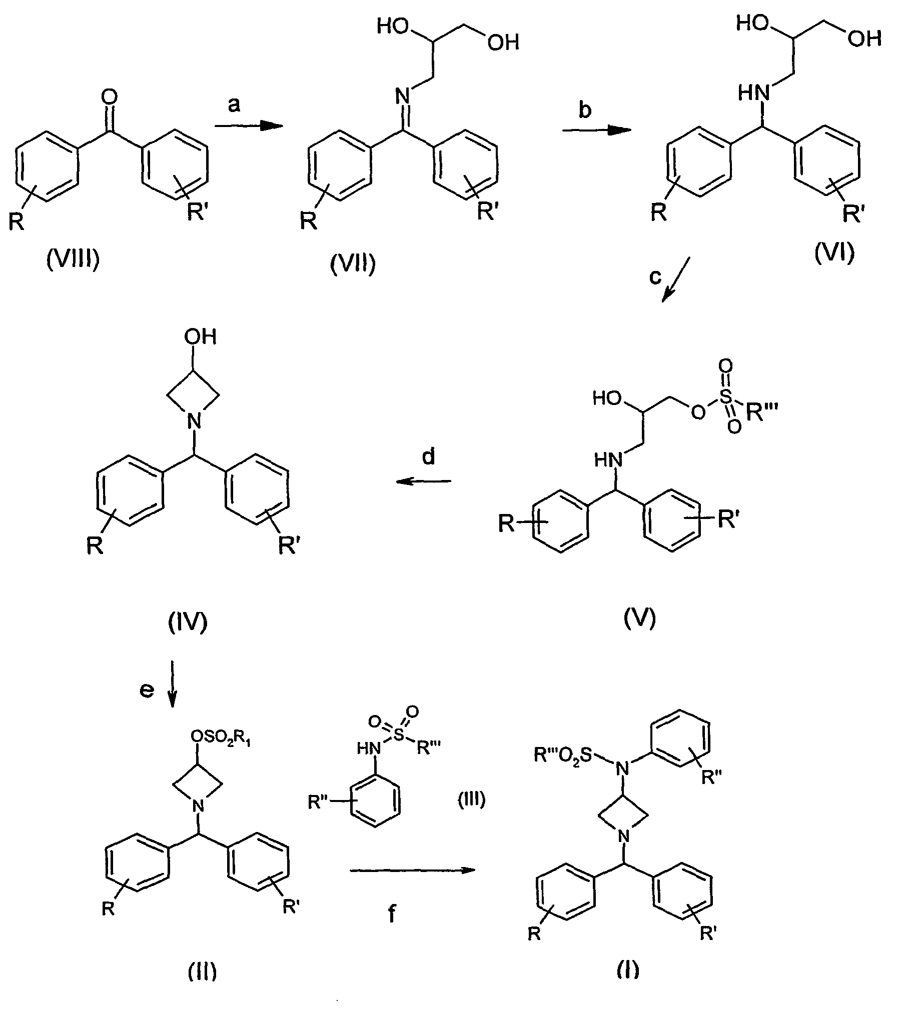
a) une cétone de formule (VIII) est condensée avec du 3-amino-1,2-propanediol, en présence ou non d'un solvant, en présence ou non d'un catalyseur acide ou basique, à une température comprise entre 0°C et 150°C ;
b) l'imine diol de formule (VII) obtenue à l'étape a) et éventuellement non isolée est réduite avec un hydroborure ou de l'hydrogène gazeux, en présence d'un catalyseur métallique dans un solvant pour former l'amine diol de formule (VI) ;
c) l'amine diol de formule (VI) obtenu à l'étape b) et éventuellement non isolé est activé par un agent sulfonylant en présence d'une base, dans un solvant et éventuellement un co-solvant à une température allant de -78°C à 40° pour former un ester sulfonique de formule (V) éventuellement non isolé;
d) l'ester sulfonique de formule (V) obtenu à l'étape c) et éventuellement non isolé est cyclisé par chauffage dans un solvant en présence ou non d'une base, entre 0°C et 130°C pour former l'azétidinol de formule (IV) et est éventuellement isolé sous forme de sel ;
e) l'azétidinol de formule (IV) obtenu à l'étape d) est transformé en sulfonate de formule (II) par action d'un agent sulfonylant en présence d'une base, dans un solvant, à une température comprise entre -78° et +40°C ;
f) le sulfonate obtenu à l'étape e) est condensé avec un sulfonamide de formule (III) en présence d'une base, dans le même solvant que celui utilisé à l'étape e), pour former le composé de formule (I) ;
Dans les formules (I), (II), (III), (IV), (V), (VI), (VII) et (VIII), R , R' et R" représentent indépendamment l'un de l'autre, un ou plusieurs radicaux hydrogène, halogène (CI, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy ; R"' représente un groupe alkyle ou perfluoroalkyle linéaire ou ramifié de 1 à 6 atomes de carbone ou un groupement aryle éventuellement substitué par un ou plusieurs radicaux R" ; R1 représente un groupe méthyle, CF₃, C₄F₉, C₈F₁₇ ou phényle éventuellement substitué par un groupe méthyle, halogèno ou nitro.

2. Procédé de synthèse selon la revendication 1 **caractérisé en ce que** dans l'étape a) le solvant est le xylène et le catalyseur de l'acide paratoluène sulfonique.

3. Procédé de synthèse selon la revendication 1 **caractérisé en ce que** dans l'étape d) le solvant est le toluène ou le xylène, la base est le NaHCO₃ ou le Na₂CO₃ et la température varie de 0 à 130 °C, de préférence entre 40 et 110°C.

4. Procédé de synthèse selon la revendication 1 **caractérisé en ce que** dans l'étape c) le solvant est de la pyridine contenant éventuellement du toluène avec du chlorure de para-toluènesulfonyle à une température allant de -78°C à +40°C, de préférence entre -30°C à +20°C.

5. Procédé de synthèse selon la revendication 1 **caractérisé en ce que** dans l'étape c) le solvant et le co-solvant sont le toluène et la pyridine dans des proportions 4 : 1 avec du chlorure de para-toluènesulfonyle à une température allant de -30°C à +20°C.

6. Procédé selon la revendication 1 **caractérisé en ce que** dans l'étape b) l'hydroborure alkalin est le NaBH₄ et le solvant est un mélange eau/éthanol à 60°C.

7. Procédé de synthèse selon la revendication 1 **caractérisé en ce que** le produit issu de l'étape d) est le bromhydrate de 1-[bis-(4-chlorophényl)méthyl]azétidin-3-ol.

8. Procédé de synthèse selon la revendication 1 **caractérisé en ce que** le produit issu de l'étape d) est le chlorhydrate de 1-[bis-(4-chlorophényl)méthyl]azétidin-3-ol.

9. Procédé de synthèse selon la revendication 1 **caractérisé en ce que** le produit issu de l'étape d) est le tétrafluoroborate de 1-[bis-(4-chlorophényl)méthyl]azétidin-3-ol.

10. Procédé de synthèse selon la revendication 1 **caractérisé en ce que** le produit issu de l'étape a) est le 3-{[Bis-(4-chloro-phényl)-méthytene]-amino}-propane-1,2-diol.

11. Procédé de synthèse selon la revendication 1 **caractérisé en ce que** le produit issu de l'étape b) est le 3-{[bis-(4-chloro-phényl)-méthyl]-amino}-propane-1,2-diol.

12. Procédé de synthèse selon la revendication 1 **caractérisé en ce que** le produit issu de l'étape c) est le toluène-4-sulfonique acide 3-{[bis-(4-chloro-phenyl)-methyl]-amino}-2-hydroxypropyl ester.

13. 3-{[Bis-(4-chloro-phenyl)-methylene]-amino}-propane-1,2-diol ; 3-{[bis-(4-chloro-phényl)-methyl]-amino}-propane-1,2-diol ; toluène-4-sulfonique acide 3-{[bis-(4-chloro-phenyl)-methyl]-amino}-2-hydroxy-propyl ester.

14. Chlorhydrate de 1-[bis-(4-chlorophényl)méthyl]azétidin-3-ol ; tétrafluoroborate de 1-[bis-(4-chlorophényl)méthyl]azétidin-3-ol.

## Claims

1. Process for synthesizing azetidine derivatives of formula (I), **characterized in that** it comprises the following steps: 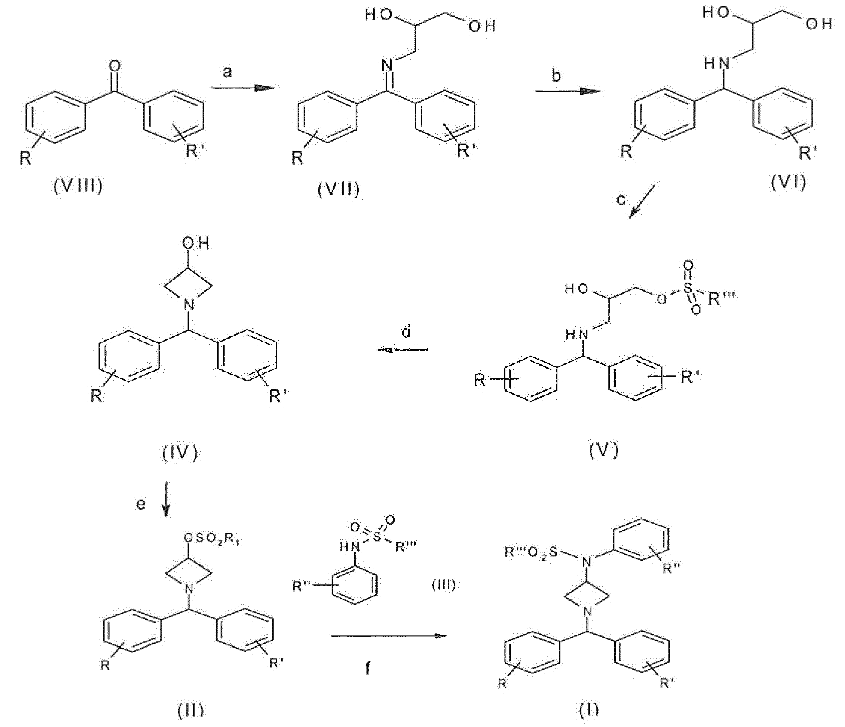
a) a ketone of formula (VIII) is condensed with 3-amino-1,2-propanediol, in the presence or absence of a solvent, in the presence or absence of an acidic or basic catalyst, at a temperature of between 0°C and 150°C;
b) the imine diol of formula (VII) obtained in step a), and optionally not isolated, is reduced with a borohydride or hydrogen gas, in the presence of a metal catalyst in a solvent so as to form the amine diol of formula (VI);
c) the amine diol of formula (VI) obtained in step b), and optionally not isolated, is activated with a sulphonylating agent in the presence of a base, in a solvent and optionally a cosolvent at a temperature ranging from -78°C to 40°C so as to for a sulphonic ester of formula (V), optionally not isolated;
d) the sulphonic ester of formula (V) obtained in step c), and optionally not isolated, is cyclized by heating in a solvent in the presence or absence of a base, and between 0°C and 130°C, so as to form the azetidinol of formula (IV), and is optionally isolated in the form of a salt;
e) the azetidinol of formula (IV) obtained in step d) is converted to the sulphonate of formula (II) through the action of a sulphonylating agent in the presence of a base, in a solvent, at a temperature of between -78° and +40°C;
f) the sulphonate obtained in step e) is condensed with a sulphonamide of formula (III) in the presence of a base, in the same solvent as that used in step e), so as to form the compound of formula (I);
in formulae (I), (II), (III), (IV), (V), (VI), (VII) and (III), R, R' and R" represent, independently of one another, one or more hydrogen, halogen (Cl, F, Br, I), cyano or nitro radicals, linear or branched alkyl radicals containing from 1 to 6 carbon atoms, linear or branched alkoxy radicals containing from 1 to 6 carbon atoms, linear or branched alkyl carboxylate radicals, wherein the alkyl contains from 1 to 6 carbon atoms, trifluoromethyl trifluoromethoxy radicals; R"' represents a linear or branched alkyl or perfluoroalkyl group containing from 1 to 6 carbon atoms or an aryl group optionally substituted with one or more R" radicals; and R1 represents a methyl group, CF₃ group, C₄F₉ group, C₈F₁₇ group or phenyl group optionally substituted with a methyl, halo or nitro group.

2. Synthetic process according to Claim 1, **characterized in that**, in step a), the solvent is xylene and the catalyst is para-toluenesulphonic acid.

3. Synthetic process according to Claim 1, **characterized in that**, in step d), the solvent is toluene or xylene, the base is NaHCO₃ or Na₂CO₃. and the temperature ranges from 0 to 130°C, preferably between 40 and 110°C.

4. Synthetic process according to Claim 1, **characterized in that**, in step c), the solvent is pyridine optionally containing toluene with para-toluenesulphonyl chloride at a temperature ranging from -78°C to +40°C, preferably between -30°C and +20°C.

5. Synthetic process according to Claim 1, **characterized in that**, in step c), the solvent and the cosolvent are toluene and pyridine in proportions of 4:1 with para-toluenesulphonyl chloride at a temperature ranging from -30°C to +20°C.

6. Process according to Claim 1, **characterized in that**, in step b), the alkali metal borohydride is NaBH₄ and the solvent is a water/ethanol mixture at 60°C.

7. Synthetic process according to Claim 1, **characterized in that** the product derived from step d) is 1-[bis(4-chlorophenyl)methyl]azetidin-3-ol hydrobromide.

8. Synthetic process according to Claim 1, **characterized in that** the product derived from step d) is 1-[bis(4-chlorophenyl)methyl]azetidin-3-ol hydrochloride.

9. Synthetic process according to Claim 1, **characterized in that** the product derived from step d) is 1-[bis(4-chlorophenyl)methyl]azetidin-3-ol tetrafluoroborate.

10. Synthetic process according to Claim 1, **characterized in that** the product derived from step a) is 3-{[bis(4-chlorophenyl)methylene]amino}propane-1,2-diol.

11. Synthetic process according to Claim 1, **characterized in that** the product derived from step b) is 3-{[bis(4-chlorophenyl)methyl]amino}propane-1,2-diol,

12. Synthetic process according to Claim 1, **characterized in that** the product derived from step c) is 4-toluenesulphonic acid 3-{[bis(4-chlorophenyl)methyl]amino}-2-hydroxypropyl ester.

13. 3-{[bis(4-chloropheny)methylene]amino}propane-1,2-diol; 3-{[bis(4-chlorophenyl)methyl]amino}propane-1,2-diol; 4-toluenesulphonic acid 3-{[bis(4-chlorophenyl)methyl]amino}-2-hydroxypropyl ester.

14. 1-[bis(4-chlorophenyl)methyl]azetidin-3-ol hydrochloride; 1-[bis(4-chlorophenyl)methyl]azetidin-3-ol tetrafluoroborate.

## Patentansprüche

1. Verfahren zur Synthese von Azetidinderivaten der Formel (I), **dadurch gekennzeichnet, dass** es die folgenden Schritte umfasst: 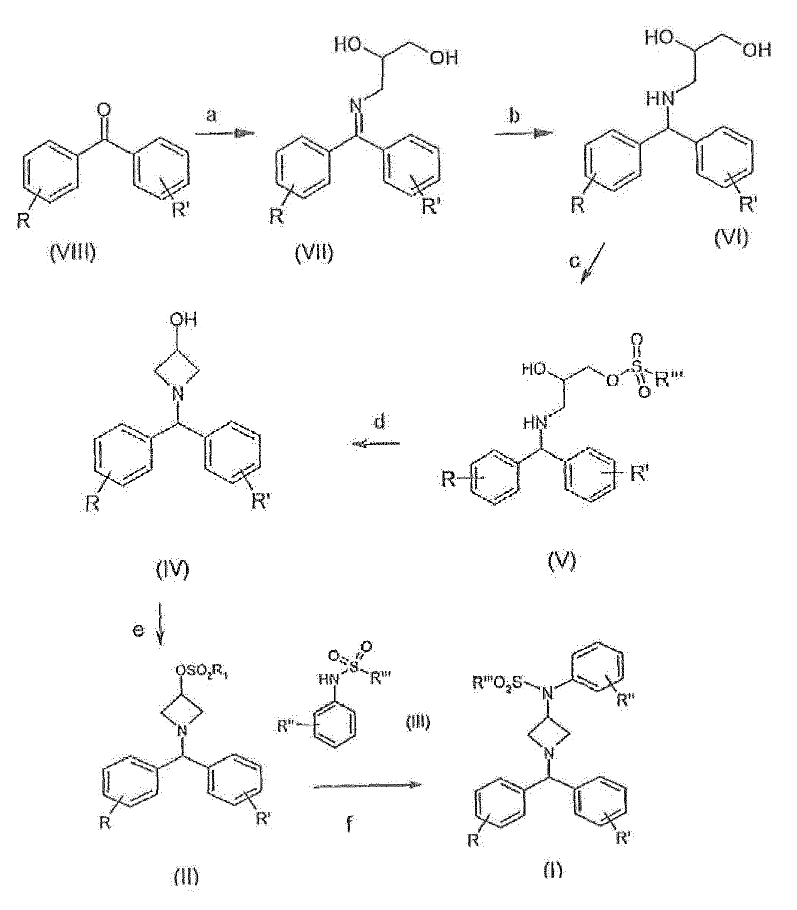
a) ein Keton der Formel (VIII) wird in Gegenwart oder Abwesenheit eines Lösungsmittels und in Gegenwart oder Abwesenheit eines sauren oder basischen Katalysators bei einer Temperatur zwischen 0°C und 150°C mit 3-Amino-1,2-propandiol kondensiert;
b) das in Schritt a) erhaltene und gegebenenfalls nicht isolierte Imindiol der Formel (VII) wird mit einem Borhydrid oder gasförmigem Wasserstoff in Gegenwart eines Metallkatalysators in einem Lösungsmittel zu dem Amindiol der Formel (VI) reduziert;
c) das in Schritt b) erhaltene und gegebenenfalls nicht isolierte Amindiol der Formel (VI) wird in Gegenwart einer Base in einem Lösungsmittel und gegebenenfalls einem Cosolvens bei einer Temperatur von -78°C bis 40°C mit einem Sulfonylierungsmittel aktiviert, was einen gegebenenfalls nicht isolierten Sulfonsäureester der Formel (V) ergibt;
d) der in Schritt c) erhaltene und gegebenenfalls nicht isolierte Sulfonsäureester der Formel (V) wird durch Erhitzten in einem Lösungsmittel in Gegenwart oder Abwesenheit einer Base zwischen 0°C und 130°C cyclisiert, was das Azetidinol der Formel (IV) ergibt, das gegebenenfalls in Salzform isoliert wird;
e) das in Schritt d) erhaltene Azetidinol der Formel (IV) wird durch Einwirkung eines Sulfonylierungsmittels in Gegenwart einer Base in einem Lösungsmittel bei einer Temperatur zwischen -78°C und +40°C in das Sulfonat der Formel (II) umgewandelt;
f) das in Schritt e) erhaltene Sulfonat wird in Gegenwart einer Base in dem gleichen Lösungsmittel wie dem in Schritt e) verwendeten mit einem Sulfonamid der Formel (III) zu der verbindung der Formel (I) umgewandelt;
wobei in den Formel (I), (II), (III), (IV), (V), (VI), (VII) und (VIII) R, R' und R'' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Carboxylatreste mit 1 bis 6 Kohlenstoffatomen, Trifluormethylreste, Trifluormethoxyreste stehen; R''' für eine lineare oder verzweigte Allyl- oder Perfluoralkylgruppe mit 1 bis 6 Kohlenstoffatomen oder eine gegebenenfalls durch einen oder mehrere Reste R'' subsituierte Arylgruppe steht; R1 für eine Methylgruppe, eine CF₃-Gruppe, eine C₄F₉-Gruppe, eine C₈F₁₇-Gruppe oder eine gegebenenfalls durch eine Ethyl-, Halogen- oder Nitrogruppe subsituierte Phenylgruppe steht.

2. Syntheseverfahren nach Anspruch 1, **dadurch gekennzeichnet, dass** es sich in Schritt a) bei dem Lösungsmittel um Xylol und bei dem Katalysator um p-Toluolsulfonsäure handelt.

3. Syntheseverfahren nach Anspruch 1, **dadurch gekennzeichnet, dass** es sich in Schritt d) bei dem Lösungsmittel um Toluol oder Xylol und bei der Base um NaHCO₃ oder Na₂CO₃ handelt und die Temperatur von 0 bis 130°C, vorzugsweise zwischen 40 und 110°C, variiert.

4. Syntheseverfahren nach Anspruch 1, **dadurch gekennzeichnet, dass** es sich in Schritt c) bei dem Lösungsmittel um Pyridin, das gegebenenfalls Toluol enthält, mit para-Toluolsulfonylchlorid bei einer Temperatur von -78°C bis +40°C, vorzugsweise zwischen -30°C und +20°C, handelt.

5. Syntheseverfahren nach Anspruch 1, **dadurch gekennzeichnet, dass** es sich in Schritt c) bei dem Lösungsmittel und dem Cosolvens um Toluol und Pyridin in den Anteilen 4 : 1 mit para-Toluolsulfonylchlorid bei einer Temperatur von -30°C bis +20°C, handelt.

6. Syntheseverfahren nach Anspruch 1, **dadurch gekennzeichnet, dass** es sich in Schritt b) bei dem Alkalimetallborhydrid um NaBH₄ und bei dem Lösungsmittel um eine Wasser/Ethanol-Mischung bei 60°C handelt.

7. Syntheseverfahren nach Anspruch 1, **dadurch gekennzeichnet, dass** es sich bei dem Produkt von Schritt d) um 1-[Bis(4-chlorphenyl)methyl]-azetidin-3-ol-hydrobromid handelt,

8. Syntheseverfahren nach Anspruch 1, **dadurch gekennzeichnet, dass** es sich bei dem Produkt von schritt d) um 1-[Bis(4-chlorphenyl)methyl]-azetidin-3-ol-hydrochlorid handelt.

9. Syntheseverfahren nach Anspruch 1, **dadurch gekennzeichnet, dass** es sich bei dem Produkt von Schritt d) um 1-[Bis(4-chlorphenyl)methyl]-azetidin-3-ol-tetrafluoroborat handelt.

10. Syntheseverfahren nach Anspruch 1, **dadurch gekennzeichnet, dass** es sich bei dem Produkt von Schritt a) um 3-{[Bis(4-chlorphenyl)methylen]-amino}propan-1,2-diol handelt.

11. Syntheseverfahren nach Anspruch 1, **dadurch gekennzeichnet, dass** es sich bei dem Produkt von Schritt b) um 3-{[Bis(4-chlorphenyl)methyl]amino}-propan-1,2-diol handelt.

12. Syntheseverfahren nach Anspruch 1, **dadurch gekennzeichnet, dass** es sich bei dem Produkt von Schritt c) zum 4-Toluolsulfonsäure-3-{[bis(4-chlorphenyl)methyl]amino}-2-hydroxypropylester handelt.

13. 3-{[Bis(4-chlorphenyl)methylen]amino}propan-1,2-diol, 3-{[Bis(4-chlorphenyl)methyl]amino}propan-1,2-dipol; 4-Toluolsulfonsäure-3-{[bis(4-chlorphenyl)methyl]amino)-2-hydroxypropylester.

14. 1-[Bis(4-chlorphenyl)methyl]azetidin-3-ol-hydrochlorid; 1-[Bis(4-chlorphenyl)methyl]azetidin-3-ol-tetrafluoroborat.
